# EUROPÄISCHE PATENTSCHRIFT

(11) **EP 0 481 365 B1**
(45) Veröffentlichungstag und Bekanntmachung des Hinweises auf die Patenterteilung: **22.06.1994**
(21) Anmeldenummer: 91117330.0
(22) Anmeldetag: 11.10.1991
(51) Int. Cl.: A61F 2/04

(54) **Vorrichtung zum Aufweiten einer Stenose in einer Körperröhre**
Device for expanding a stenosis in a body duct
Dispositif pour dilater une sténose dans un conduit corporel

(30) Priorität: 13.10.1990 DE 9014230 U
(43) Veröffentlichungstag der Anmeldung: 22.04.1992
(60) Teilanmeldung: 93117016.1
(73) Patentinhaber: ANGIOMED AG, 76227 Karlsruhe (DE)
(72) Erfinder: Schnepp-Pesch, Wolfram, W-7505 Ettlingen (DE); Lindenberg, Josef, W-7500 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 119 688
- EP-A- 0 282 175
- EP-A- 0 380 668
- WO-A-90/04982
- FR-A- 2 257 262
- FR-A- 2 617 721
- US-A- 3 657 744
- US-A- 3 868 956
- US-A- 4 955 859

## Beschreibung

Die Erfindung betrifft eine Vorrichtung zum Aufweiten einer Stenose in einer Körperröhre nach dem Oberbegriff des Anspruchs 1. Eine solche Vorrichtung ist aus der EP-A-0 380 668 oder der US-A-3 868 956 oder der FR-A-2 617 721 bekannt.

Ein Verschluß in einem Gefäß, wie in einer Arterie, wird zunächst in der Regel erweitert, indem Ablagerungsmaterial, wie Plaques entfernt werden, beispielsweise durch Herausschneiden und Absaugen oder dergleichen. Dies kann aber nicht bis zum Gewebematerial geschehen, da hier eine Verletzungsgefahr besteht. Wenn noch kein völliger Verschluß, sondern lediglich eine Verengung, eine Stenose, gebildet ist oder ein gewisser Durchflußbereich bei vorherigem vollständigen Verschluß in der vorgenannten Weise geschaffen wurde, ist daher eine radiale Aufweitung der Stenose gewünscht. Eine solche wird nicht nur in Blutgefäßen, sondern auch in anderen Körpergängen oder -röhren, wie im Harnleiter, Gallengang oder dergleichen, vorgenommen.

Hierzu wurde bisher ein sogenannter Ballonkatheter mit einem doppelten Lumen verwendet, der eine momentane radiale Aufweitung bewirkt, wobei diese verbleiben sollte, wenn der Ballonkatheter wieder entfernt wurde. Es wurden auch schon permanente Aufweitungen einer solchen Stenose bekannt. So wurde vorgeschlagen, auf einem derartigen Ballonkatheter ein Aufweiteteil axial fest aufzusetzen, mit dem Katheter einzuführen und durch Aufweiten des Ballonkatheters plastisch derart zu verformen, daß die radialen Abmessungen des Aufweiteteils sich vergrößern, dieses in die Wand der Stenose eingedrückt wird und nach Ablassen der Luft aus dem Ballonkatheter dieser entfernt werden kann, während das Aufweiteteil in plastisch verformtem Zustand an seinem Ort liegenbleibt. Als solches Teil wurde beispielsweise eine Manschette in Form einer "schraubenförmigen" Hülse vorgesehen.

Ein wesentlicher Nachteil des letztgenannten Verfahrens besteht darin, daß die Manschette auf dem Außenumfang eines Katheters aufsitzend in die Stenose eingebracht werden kann. Zunächst ist die axiale Festlegung aufwendig und entweder nicht zuverlässig zu erreichen, so daß das Teil beim Vorschieben des Katheters zurückbleiben kann oder aber ein späteres Lösen des Teils vom Katheter ist schwierig. Darüber hinaus kann ein außen auf einem Katheter sitzendes Aufweiteteil auch zu Beschädigungen führen. Schließlich muß die Aufweitung durch den Ballonkatheter bei einem derartigen Teil zunächst zu einer Radialerstreckung erfolgen, die wesentlich über der letztendlich wünschenswerten und zu erreichenden Radialerstrekkung liegt, da derartige Teile, wie herkömmliche biokompatible Materialien, soweit sie eine ausreichende Festigkeit haben, um eine Stenose offen zu halten, einen erheblichen elastischen Verformungsbereich aufweisen, bevor eine bleibende plastische Verformung auf dem gewünschten Radius eintritt. Dies gilt insbesondere für ein, wie erwähnt, "schraubenförmig" geformtes Teil.

Die EP-A-380 668 zeigt eine gattungsgemäße Vorrichtung mit Stents aus Memory-Metallegierung unter anderem in Form einer Schraubenwindung aus Flachdraht bzw. eines Kreuzgewebes. Bei Körpertemperatur weist die Vorrichtung ihre expandierte Stellung auf.

Aus der FR-A-26 17 721 ist ebenfalls eine grundsätzlich gattungsgemäße Vorrichtung aus einer Formgedächtnislegierung in Schraubenform entnehmbar, wobei hier allerdings die Vorrichtung bei über der Körpertemperatur liegender Temperatur ihre Konfiguration geringen Querschnitts hat und sich bei Reduzierung der Temperatur auf Körpertemperatur in die aufgeweitete Stellung aufweitet.

Eine grundsätzlich gattungsgemäße, schraubenförmige Vorrichtung ist auch aus der US-A-3 868 956 bekannt, wobei diese zur Aufweitung am Implantationsort intrinsisch durch Ohm'sche Erwärmung aufgeweitet werden soll und hierzu entweder einen durch das Innere geführten Rückleiter aufweist oder aber doppelwendelartig ausgebildet ist, wobei sich dann die Frage der Isolierung der beiden Wendelteile stellt. Auch ist unklar, wie das Material- bei Aufweitung nur durch externe Erwärmung nach Beendigung der Erwärmung in seiner aufgeweiteten Stellung verharren soll.

Es kann erforderlich werden, eine derartige Vorrichtung oder einen Stent wieder zu entfernen. Dies ist bei den Vorrichtungen nach dem Stand der Technik nur schwierig möglich.

Der Erfindung liegt daher die Aufgabe zugrunde, Vorrichtungen der eingangs genannten Art dahingehend auszubilden, daß sie einfach und leicht wieder entfernt werden können.

Erfindungsgemäß wird die genannte Aufgabe bei einem Stent der eingangs genannten Art dadurch gelöst, daß an mindestens einem Ende des Drahtteils eine Kugel ausgebildet ist. Durch ein solches Greifteil am Ende des einstückigen Drahtes kann dieser leicht mittels eines durch einen Katheter eingeführten Griffteils, wie einer Zange, eines Hakens oder dergleichen, entfernt werden. Nach Angreifen und Ziehen an der erweiternden Kugel wird der den Stent bildende Draht gestreckt, damit aufgezogen und kann so aus dem Gefäß herausgezogen werden.

Das Drahtteil wird erfindungsgemäß derart vorgeformt, daß es bei einer unterhalb der Körpertemperatur liegenden Übergangstemperatur und damit also bei der Körpertemperatur eine Form einnimmt, die der gewünschten Aufweitung der Körperröhre entspricht, während sie bei einer Temperatur unterhalb der Übergangstemperatur einen reduzierten Durchmesser aufweist und derart durch einen Katheter zum Positionierungsort eingeführt werden kann. Eine bevorzugte Ausgestaltung sieht dabei vor, daß die Formgedächtnis-Legierung eine Nickel-Titan-Legierung ist. Weiterhin können aber auch Cu-Zn-Al- oder Cu-Al-Ni-Legierungen verwendet. Derartige Legierungen sind unter den Warenzeichen Nitinol, Biometall (Firma Toki, Japan) oder Memotal bekannt.

Die die Formänderung bewirkende martensitische Reaktion bzw. Umwandlung erfolgt durch Temperaturänderung. Während die Übergangstemperatur in weiten Bereichen liegen kann, soweit sie grundsätzlich unter der Körpertemperatur liegt, sieht eine bevorzugte Ausgestaltung vor, daß die Übergangstemperatur bei 20°C liegt. Diese Temperatur ist hinreichend unterhalb der Temperatur, um eine zuverlässige Aufweitung des Teils aus Formgedächtnis-Legierung in seinen Hochtemperaturzustand zu bewirken. Gleichzeitig liegt die Temperatur so hoch, daß ein vom Operateur bei Umgebungstemperatur genommenes Teil, das damit in seinem Niedertemperaturzustand mit geringen Radialabmessungen ist, sich erst nach einer gewissen Zeit radial aufweitet, die ausreichend ist, um das Teil in den Körper und bis zu der Stenose einzuführen.

Die Verkürzung des Teils bei einer radialen Aufweitung kann durch eine von vorneherein vorgesehene größere Länge berücksichtigt werden.

Es sind verschiedene Ausgestaltungen und Ausbildungen derartiger Vorrichtungen möglich. So kann vorgesehen sein, daß das Teil aus Formgedächtnis-Legierung ein aus Draht bestehendes Teil ist.

Letzteres gilt auch für weitere bevorzugte Ausgestaltungen, bei denen vorgesehen ist, daß das Teil aus einem Metallgeflecht oder -gestricke besteht und als Zylindermantel ausgebildet ist oder daß das Teil aus Formgedächtnis-Legierung aus einem mehrfach schraubenförmig geführten Draht gebildet ist.

Eine äußerst bevorzugte Ausgestaltung der erfindungsgemäßen Vorrichtung zeichnet sich dadurch aus, daß das Metallmaterial, in axialer Richtung, mäanderförmig ausgebildet ist und die einzelnen Mäander nahezu ringförmig gebogen sind. Wenn, der Mäander in axialer Richtung Ausbiegungen aufweist, so sieht ein bevorzugte Weiterbildung vor, daß die Ausbiegungen eines Mäanderschenkels mit einem benachbarten Mäanderschenkel verbunden ist. Die Verbindung kann insbesondere durch Löten erfolgen. Wenn nicht sämtliche Ausbiegungen mit dem benachbarten Mäanderschenkel in der typischen Weise fest verbunden sind, sondern lediglich ein Teil derselben, so kann gemäß einer weiteren bevorzugten Ausgestaltung vorgesehen sein, daß die Verbindungskräfte der Verbindungen größer sind als die Wiegekräfte der Mäanderbiegungen. Hierdurch kann die erfindungsgemäße Vorrichtung, die auch als Endoprothese oder Stent zu bezeichnen ist, in den Bereichen, in denen die Mäander in axialer Richtung nicht durch Verbindungen, wie Lötstellen, fest miteinander verbunden sind gedehnt oder gestaucht und damit in ihrer Länge angepaßt werden, ohne daß die an anderen Stellen vorhandenen Verbindungen (Lötungen) reißen oder brechen.

Andererseits sollen die Verbindungsstellen als definierte Bruchstellen ausgebildet sein und zwar dann, wenn eine in der vorbeschriebenen Weise ausgebildete Endoprothese später wieder entfernt werden soll, so daß beim Ziehen am einen Ende des die Endoprothese bildenden Drahtes die Verbindungsstellen aufbrechen, der Draht sich streckt und daher in bequemer Weise die Endoprothese entfernt werden kann. Das Entfernen einer Endoprothese in der vorstehend beschriebenen Ausgestaltung, aber auch in rundgestrickter Ausgestaltung oder bei einer wendelförmig geformten Endoprothese wird erleichtert, wenn, an mindestens einem Ende des die Vorrichtung bildenden Drahtes eine Kugel ausgebildet ist. Es kann dann an der Kugel mit einer Hohlzange angegriffen werden und die Strekkung der Endoprothese erreicht werden.

Während die Außenkontur der Endoprothesen in der Regel zylindermantelförmig ist, sehen in gewissen Einsätzfällen äußerst bevorzugte Ausgestaltungen konusförmige oder doppelkonusförmige Außenkonturen vor.

Eine weitere bevorzugte Ausgestaltung sieht vor, daß das Metall des insoweit beschriebenen Stents in gewebekompatible Kunststoffe oder vorzugsweise Silikon eingebettet ist, so daß trotz der Zwischenräume zwischen Bereichen des Memorymetalls ein geschlossener Mantelbereich geschaffen wird. Hierdurch wird beim malignen Tumoren verhindert, daß Zellen ins Innere der Endoprothese einwachsen.

Die erfindungsgemäße Endoprothese kann in vielfältigen Bereichen und zu vielfältigen Zwecken eingesetzt werden, so in der Urethra, im Ureter, im Harnblasenhals, in Dedukti Bilipheri, in Blutgefäßen, wie Arterien und Venen, im Ösophagus und in der Trachea sowie im Darmbereich, insbesondere im Intestinum Rektum.

Die Erfindung bietet steife und hochflexible, insbesondere um ihre Achse biegbare Endoprothesen an, letzteres gilt insbesondere für Endoprothesen mit mäanderförmig geführtem Draht.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnungen im einzelnen erläutert sind. Dabei zeigt:
- Figur 1: eine Ausgestaltung mit einem schraubenförmig gewickelten Draht;
- Figur 2: das Maschenmuster eines erfindungsgemäßen rundgestrickten Stents;
- Figur 3: eine schematische Darstellung eines Stents mit mäanderförmig geführtem Draht, wobei die einzelnen Mäander teilringförmig gebogen sind, mit zylindrischer Außenkontur; und
- Figur 4: eine Endoprothese ähnlich der der Figur 6 mit doppelkonischer Außenkontur war ebenfalls in schematischer Darstellung;

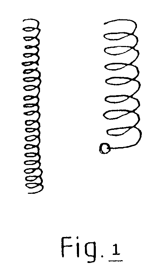

In der Figur 1 ist in einer ersten Ausgestaltung einer erfindungsgemäßen Vorrichtung zum Aufweiten einer Stenose in Körperröhren, wie in einer Arterie, im Harnleiter, im Gallengang oder dergleichen, im folgenden als "Stent" bezeichnet, dargestellt. Der Stent der Figur 1 weist Schrauben oder Wendelform auf. Die erfindungsgemäßen Stents bestehen aus Metall mit einem Formgedächtnis, insbesondere aus Nicekl-Titan-Legierungen, aber auch aus Cu-Zn-Al- oder Cu-Al-Ni. Formgedächtnislegierungen sind unter den Bezeichnungen Nitinol, Bimetall oder Memotal bekannt. Die Herstellung des Stents geschieht derart, daß der Stent zunächst in seine Hochtemperaturform der Figur 1, vorgeformt wird, anschließend einer Wärmebehandlung unterzogen wird. Nach Abkühlung unter die Übergangstemperatur wird der Stent bleibend in seine querschnittreduzierte Niedertemperaturform gebracht. Wenn er über die Übergangstemperatur, die im Bereich von 30 bis 35 °C, vorzugsweise bei 35°C liegt, wieder erwärmt wird, so "erinnert" er sich an seine Hochtemperaturform und nimmt diese wieder an.

Bei der radialen Aufweitung verkürzt sich der Stent der Figur 1. Diese Verkürzung kann aber durch entsprechende Längenwahl berücksichtigt werden. Das Ende weist eine kugelförmige Erweiterung 16 zum Entfernen auf.

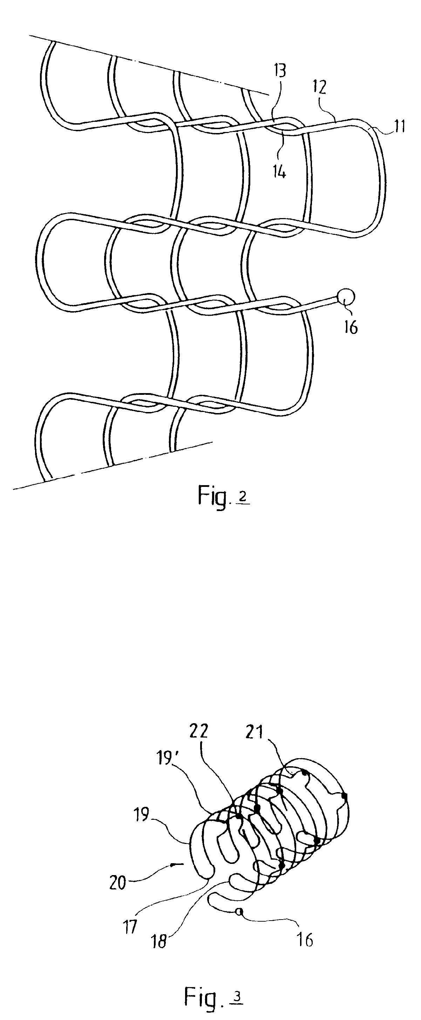

In der Figur 2 ist das Maschenbild einer erfindungsgemäβen rundgestrickten Endoprothese dargestellt. Sie weist übliche Strickmaschen aus einem Schußfaden 11 auf, wobei die Maschen eines Schenkel 12, einen Kopf 13 an ihrer oberen Bindungsstelle und einen Fuß 14 an der unteren Bindungsstelle aufweisen, um den der Kopf herumgelegt ist.

Am Ende des Drahtfadens 11 ist auch hier eine Kugel 16 befestigt. Wenn eine eingelegte Endoprothese (Stent) aus irgendwelchen Gründen wieder entfernt werden soll, so kann mittels einer Hohlzange an der Kugel 16 angegriffen und gezogen werden, wodurch das Maschenbild der Endoprothese aufgezogen und diese derart aus dem Körperhohlraum , indem sie plaziert war, entfernt werden kann.

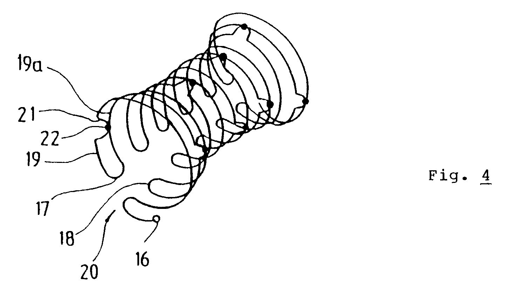

Ein solches Entfernen durch Strecken des die jeweilige Endoprothese bildenden Drahtfadens ist auch bei den Ausgestaltungen der Figuren 3 und 4 möglich. Diese Endoprothesen bestehen aus mäanderförmig in Längsrichtung gewundenen Draht (die Mäander selbst erstrecken sich also quer zur Längsrichtung), wobei die Mäander ringförmig gebogen sind, so daß die einander abgewandten Stege benachbarter Mäander aufeinander zugebogen sind. Eine derartige Endoprothese weist den wesentlichen Vorteil auf, daß sie in ihrer Länge verändert werden kann, ohne daß hierdurch der Querschnitt wesentlich beinflusst wird. Die Endoprothese kann gegenüber ihrer Normallängserstreckung gestaucht oder gestreckt werden, wobei sie in der so erfolgten Stellung durch die Reibkräfte zwischen ihr und den umgebenden Gefäßwandungen gehalten wird.

In bevorzugter Ausgestaltung weisen Schenkel 19 der Mäander 20 Ausbiegungen 21 auf, die bis zum benachbarten Mäanderschenkel - beispielsweise 19′ - reichen. Zumindestens ein Teil der Ausbiegung 21 kann mit dem jeweils benachbarten Mäanderschenkel 19′durch eine Lötstelle 22 verbunden sein. Je nach Einsatzzweck können sämtliche Ausbiegungen mit benachbarten Mäanderschenkeln verbunden sein - in diesem Falle ist die Länge der Endoprothese begrenzt; es können lediglich ein Teil der Ausbiegungen verbunden sein, während an einigen Ausbiegungen keine Lötstellen vorhanden sind - hierdurch kann die Länge in begrenztem Umfange verändert werden; schließlich können Endoprothesen derart ausgebildet sein, daß keinerlei Ausbiegungen mit benachbarten Mäanderschenkeln fest verbunden sind; die Ausbiegungen bedingen eine gewisse Nachgiebigkeit in radialer bzw. angularer Richtung der Endoprothese.

Auch die.Endoprothesen der Figuren 3 und 4 weisen wieder an den Fadenenden die Kügelchen 16 auf, um die Endoprothese durch Strecken des sie bildenden Fadens zu entfernen. Wenn Lötstellen 22 vorhanden sind, so ist deren Befestigungskraft wesentlich geringer als die Reißkraft des Fadens; sie sollte aber größer sein als die Biegekraft des Drahtes im Bereich der Stege 17, 18 der Mäanderwindungen

Wie auch andere erfindungsgemäße Endoprothesenausgestaltungen kann die vorstehend beschriebene zylindrische Außenkontur aufweisen, wie dies in Figur 3 skizziert ist; sie kann aber auch, wie auch andere der gezeigten Endoprothesen konische oder doppelkonische Gestalt aufweisen, wie dies in der Figur 4 der Fall ist, wobei hier der größere Durchmesser an den Stirnseiten der Endoprothese ausgebildet ist.

Die erfindungsgemäße Endoprothese gemäß den Figuren 3 und 4 weist eine sehr hohe Flexibilität auf, die auch höher liegt, als die des Gegenstands der Figur 2. Die Endoprothese der Figuren 3 und 4 ist praktisch vollständig biegbar. Die erfindungsgemäße Metall-Endoprothesen können in kompatible Kunststoffe oder vorzugsweise Silikon eingebettet werden, welche die gleiche Kontur wie der Stent selbst annimmt. Hierdurch kann verhindert werden, daß im Bereich einer derart augestalteten Endoprothese ein maligner Tumor durch den Durchschnittsstent hindurch wandern kann.

Der vorstehende durch Mäanderführung erhaltene Stent lässt sich in vielfältiger Weise und zu vielfältigen Verwendungszwecken einsetzen. Besonders vorteilhaft lässt sich ein solcher Stent als Harnröhrenstent im Bereich der Prostata zur Aufweitung des dortigen Harnröhrenbereichs bei vergrößerter Prostata einsetzen. Er passt sich dabei insbesondere gut aufgrund seiner Flexibilität der doppelhahnförmigen Ausbildung der männlichen Harnröhre an. Durch seine Längenveränderlichkeit kann er so in der Länge verändert werden, daß sein rückwärtiges oder äußeres Ende nicht in den Bereich des externen Sphinkter zu liegen kommt, so daß der Stent dessen Funktion nicht beeinträchtigt. Insbesondere bei einer solchen Anwendung ist die oben beschriebene Möglichkeit der Extraktion des Stents durch Strecken des ihn bildenden Drahtfadens und Herausziehen äußerst vorteilhaft.

## Patentansprüche

1. Vorrichtung zum Aufweiten einer Stenose in einer Körperröhre, wie in einer Arterie, im Harnleiter, im Gallengang oder dergleichen, mit einem Drahtteil mit im wesentlichen zyliindermantelförmiger Außerkontur aus Formgedächtnis-Legierung, wobei sich das Teil bei einer Übergangstemperatur, die oberhalb der Umgebungstemperatur, aber unterhalb der Körpertemperatur liegt, radial unter Beibehaltung einer im wesentlichen zylindrischen Außenkontur aufweitet, wobei der Durchmesser bei einer Temperatur unterhalb der Übergangstemperatur unter dem Durchmesser des Gefäßes liegt, dadurch gekennzeichnet, daß an mindestens einem Ende des Drahtteils eine Kugel (16) ausgebildet ist.

2. Vorrichtung nach Anspruch 1, dadurch gekennzeichnet, daß das Drahtteil aus einem Metallgeflecht oder -gestricke besteht und als Zylindermantel ausgebildet ist.

3. Vorrichtung nach Anspruch 2, dadurch gekennzeichnet, daß das Gestricke ein Rundgestricke ist.

4. Vorrichtung nach Anspruch 1, dadurch gekennzeichnet, daß das Drahtteil aus Formgedächtnis-Legierung aus einem mehrfach schraubenförmig geführten Draht gebildet ist.

5. Vorrichtung nach Anspruch 1, dadurch gekennzeichnet, daß bandförmiges Metallmaterial, als Rund- oder als Flachdraht im Mäander derart geführt ist, daß jeweils benachbarte Schenkel eines Mäanders im wesentlichen in Umfangsrichtung verlaufen, während die die Schenkel verbindenden Stege im wesentlichen achsparallel gerichtet sind.

6. Vorrichtung nach Anspruch 5, dadurch gekennzeichnet, daß Mäander (20) in axialer Richtung Ausbiegungen (21) aufweist.

7. Vorrichtung nach Anspruch 6, dadurch gekennzeichnet, daß die Ausbiegungen (21) eines Mäanderschenkels (19) mit einem benachbarten Mäanderschenkel (19) verbunden ist (bei 22).

8. Vorrichtung nach Anspruch 7, dadurch gekennzeichnet, daß die Verbindungskräfte der Verbindungen (22) größer sind als die Biegekräfte der Mäanderbiegungen (17, 18).

9. Vorrichtung nach Anspruch 6 oder 7, dadurch gekennzeichnet, daß die Verbindungskräfte der Verbindungen (22) geringer als die Reißkraft des Drahtteils sind.

10. Vorrichtung nach einem der vorangehenden Ansprüche, gekennzeichnet durch zylinderförmige Außenkontur.

11. Vorrichtung nach einem der Ansprüche 1 bis 9, gekennzeichnet durch konusförmige Außenkontur.

12. Vorrichtung nach einem der Ansprüche 1 bis 9, gekennzeichnet durch doppelkonusförmige Außenkontur.

13. Vorrichtung nach einem der vorangehenden Ansprüche, dadurch gekennzeichnet, daß die Metallelemente der Vorrichtung in einen geschlossenen Mantel aus gewebekompatiblen Grundstoffen, insbesondere Silikon eingebettet sind.

14. Vorrichtung nach einem der Ansprüche 1 bis 13, dadurch gekennzeichnet, daß die Formgedächtnis-Legierung eine Nickel-Titan-Legierung ist.

15. Vorrichtung nach einem der Ansprüche 1 bis 14, dadurch gekennzeichnet, daß die Übergangstemperatur zwischen 30 und 35 °C liegt.

16. Vorrichtung nach Anspruch 15, dadurch gekennzeichnet, daß die Übergangstemperatur bei 35° C liegt.

## Claims

1. Device for expanding a stenosis in a body cavity, such as in an artery, the ureter, the bile duct, etc., having a wire part with a substantially cylindrical envelope-like outer contour made from memory alloy, in which the part expands at a transition temperature above ambient temperature, but below body temperature, in radial manner, whilst maintaining a substantially cylindrical outer contour, the diameter being smaller than the diameter of the vessel at a temperature below the transition temperature, characterized in that a ball (16) is formed at at least one end of the wire part.

2. Device according to claim 1, characterized in that the wire part is made from braided or knitted metal and is constructed as a cylindrical envelope.

3. Device according to claim 2, characterized in that the knitted part is a circular knitted part.

4. Device according to claim 1 characterized in that the memory alloy wire part is formed from a multiply helically guided wire.

5. Device according to claim 1, characterized in that strip-like metal material, in the form of a round or flat wire, is guided in meander-form in such a way that in each case adjacent legs of a meander run substantially circumferentially, whereas the webs connecting the legs are substantially axially parallel.

6. Device according to claim 5, characterized in that the meanders (20) have outward deflections (21) in the axial direction.

7. Device according to claim 6, characterized in that the outward deflections (21) of a meander leg (19) are connected to an adjacent meander leg (19) at (22).

8. Device according to claim 7, characterized in that the connecting forces of the connections (22) are higher than the bending forces of the meander bends (17, 18).

9. Device according to claim 6 or 7, characterized in that the connecting forces of the connections (22) are lower than the tensile strength of the wire part.

10. Device according to one of the preceding claims, characterized by a cylindrical outer contour.

11. Device according to one of the claims 1 to 9, characterized by a conical outer contour.

12. Device according to one of the claims 1 to 9, characterized by a double conical outer contour.

13. Device according to one of the preceding claims, characterized in that the metal elements of the device are embedded in a closed envelope of tissue-compatible basic materials, particularly silicone.

14. Device according to one of the claims 1 to 13, characterized in that the memory alloy is a nickel-titanium alloy.

15. Device according to one of the claims 1 to 14, characterized in that the transition temperature is between 30 and 35°C.

16. Device according to claim 15 characterized in that the transition temperature is 35°C.

## Revendications

1. Dispositif pour dilater une sténose dans un conduit corporel, par exemple dans une artère, dans l'uretère, dans le canal cholédoque et similaires, comprenant une partie métallique filiforme à profil extérieur sensiblement en forme de gaine cylindrique en alliage à mémoire de forme, cette partie se dilatant radialement sous l'effet d'une température de transition de résilience supérieure à la température ambiante mais inférieure à la température corporelle tout en gardant un profil extérieur essentiellement cylindrique, le diamètre étant inférieur au diamètre du vaisseau lorsque la température est inférieure à celle de la température de transition de résilience, caractérisé en ce que qu'une sphère (16) est conformée au moins à une des extrémités de la partie métallique filiforme.

2. Dispositif selon la revendication 1, caractérisé en ce que la partie métallique filiforme consiste en un treillis ou tricot métallique et en ce qu'elle est conformée en gaine cylindrique.

3. Dispositif selon la revendication 2, caractérisé en ce que le tricot est un tricot tricoté circulairement.

4. Dispositif selon la revendication 1, caractérisé en ce que la partie métallique filiforme en alliage à mémoire de forme est constituée d'un fil métallique de forme hélicoïdale.

5. Dispositif selon la revendication 1, caractérisé en ce qu'un matériau métallique en bandes tel que du fil rond ou plat est conformé en serpentin et en ce que chacune des branches adjacentes du serpentin s'étend en direction tangentielle, tandis que celles reliées par une tige de liaison sont dirigées sensiblement axialement.

6. Dispositif selon la revendication 5, caractérisé en ce que le serpentin (20) présente des courbes (21) selon la direction axiale.

7. Dispositif selon la revendication 6, caractérisé en ce que les courbes (21) d'une des branches (19) du serpentin sont reliées (en 22) a une branche (19) adjacente du serpentin.

8. Dispositif selon la revendication 7, caractérisé en ce que les forces de liaison des liaisons (22) sont supérieures aux forces de courbure des courbes du serpentin (17,18).

9. Dispositif selon la revendication 6 ou 7, caractérisé en ce que les forces de liaison des liaisons (22) sont inférieures à la force d'arrachement de la partie métallique filiforme.

10. Dispositif selon l'une quelconque des revendications précédentes, caractérisé par un profil extérieur en forme de cylindre.

11. Dispositif selon l'une quelconque des revendications 1 à 9 caractérisé par un profil extérieur conique.

12. Dispositif selon l'une quelconque des revendications 1 à 9, caractérisé par un profil extérieur biconique.

13. Dispositif selon l'une quelconque des revendications précédentes, caractérisé en ce que les éléments métalliques du dispositif sont enfermés dans un enrobage en matière compatible avec les tissus, en particulier en silicone.

14. Dispositif selon l'une quelconque des revendications 1 à 13, caractérisé en ce que l'alliage à mémoire de forme est un alliage de nickel et de titane.

15. Dispositif selon l'une quelconque des revendications 1 à 14, caractérisé en ce que la température de transition de résilience se situe entre 30 et 35° C.

16. Dispositif selon la revendication 15, caractérisé en ce que la température de transition de résilience se situe aux environs de 35° C.
